# EUROPEAN PATENT APPLICATION

(11) **EP 1 120 074 A1**
(43) Date of publication of application: **01.08.2001**
(21) Application number: 00830678.9
(22) Date of filing: 19.10.2000
(51) Int. Cl.: A47K 7/03, A46B 11/00, A46B 15/00

(54) **Cleaning device, particulary for medical use**

(30) Priority: 24.01.2000 IT MI000333
(71) Applicant: Nex Medical S.a.S. di Villa Annamaria & C., 20023 Cerro Maggiore (Milano) (IT)
(72) Inventor: Colombo, Aldo, 20023 Cerro Maggiore (Milano) (IT)
(74) Representative: Tansini, Elio Fabrizio

(57) **Abstract**

A cleaning device (1), in particular for medical use, comprises an operating element having a housing portion for a container (3) holding a cleansing and/or disinfecting substance and an active portion for carrying out the cleaning operation. The container holding the substance (4) is a hermetically sealed packet provided with a tear tab (6a) to release the substance onto the operating element itselft. The cleaning device further has a block of spongy material, preferably of foamed plastic material, and can be fully housed in a packaging structure 19.

## Description

The present invention relates to a cleaning device which is particularly adopted in the medical field to carry out cleaning/disinfection of the surgeons' hands before operations.

It is known that for hygienic reasons and for infection-and contamination-preventing purposes, all members of a surgical staff and above all those designed to directly operate on the patients' organic tissues, must undergo a careful prophylaxis involving hands' washing and disinfection. Among other things, this prophylaxis comprises use of appropriate cleaning devices, generally brushes and/or sponges, with which the medical staff must scrupulously rub their hands.

Obviously, the cleaning operation is integrated with use of a cleansing and/or disinfectant substance which, urged by the action of the cleaning device to also invade regions normally difficult to be reached, has the function of removing possible traces of dirt and eliminating the bacterial charge currently present on the skin or generally the material of the gloves to be worn by a medical operator.

The known art substantially involves two modalities for carrying out such cleaning/disinfection operations: in a first case cleaning devices to be shared out among the different members of the surgical staff can be employed. In other words, during the preparation step, several operators use the same device by turns, sprinkling it each time with a disinfectant and then carrying out cleaning/disinfection of their hands. Obviously, the cleaning device is stowed in a place where everybody can reach it (on a shelf close to a washbasin or in a cabinet, for example).

In this case some important drawbacks exist, since it is impossible to be always sure that the place where the device is stored is a sterile environment; in addition, since a single device is to be used by several people the risk of an insufficient disinfection is increased.

To at least partly obviate the above drawback, use of cleaning devices of the disposable type has been introduced, said devices having been previously packaged in a hermetically sealed container (generally a plastic packet) holding a certain amount of disinfectant; in this way the cleaning device typically comprising a sponge and/or a brush, is kept in the plastic container until its use, is drawn out of the container only at the appropriate time, is employed by a single user and is ultimately thrown away.

However, in this case too some drawbacks can be highlighted; first of all there is a waste of disinfectant when the hermetically-sealed package is opened, since the disinfectant contained in the packet is in an excess amount (exactly for the purpose of ensuring a complete immersion of the cleaning device therein). Furthermore, due to the presence of a "foreign" body within the disinfectant volume, a condition may be easily created according to which the true disinfection capability of the liquid itself decreases in time. In fact, when this package is being prepared, surely production of the cleaning device does not take place under sterile conditions, and this fact in the long run can bring to a decay of the properties of the disinfectant in which the device is dipped.

In particular, if the cleaning device comprises sponges and/or brushes where hiding of bacterial concentrations can occur, a complete elimination of these bacteria is practically impossible and they can begin to proliferate within the package again.

Under this situation, the technical task underlying the present invention is to provide a cleaning device, in particular for medical use, capable of substantially eliminating the above mentioned drawbacks; in particular it is an aim of the present invention to provide a cleaning device ensuring excellent sterility features.

Another aim of the present invention is to provide a cleaning device and a package for same that are particularly adapted for a disposable use and that at the same time offer very practical and quick use features.

A further aim of the present invention is to provide a cleaning device enabling waste of disinfectant material to be reduced to a minimum.

Finally, the present invention aims at providing a cleaning device for medical use having excellent features in terms of production costs, while at the same time achieving all the above listed advantages.

The technical task mentioned and the aims specified are substantially achieved by a cleaning device, in particular for medical use, according to the features described in one or more of the appended claims. Further features and advantages obtained with the present invention will be best understood from the following description.

Description of a preferred but not exclusive embodiment of a cleaning device in particular for medical use is given hereinafter, by way of non-limiting example, with the aid of the accompanying drawings, in which:

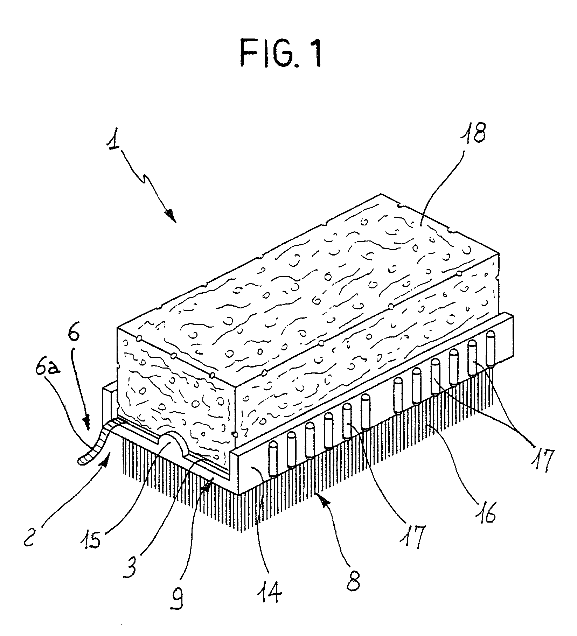

- Fig. 1 is a perspective view of the cleaning device in accordance with the present invention;
- Fig. 2 is a perspective view of the cleaning device of the invention with some components represented in an exploded view; and
- Fig. 3 shows the device referred to in Fig. 1 introduced into a packaging structure which is in accordance with the present invention as well.

With reference to the drawings, the cleaning device in accordance with the invention is generally denoted by reference numeral 1.

It essentially consists of an operating element 2 with which a container 3 for a cleansing and/or disinfectant substance 4 is associated or, more particularly, rigidly linked (by gluing, for example).

Container 3 internally has a hermetically sealed holding wall 5 which aims at retaining the cleansing substance 4; under the action of appropriate release means 6 (that may consist of a tear tab 6a) the cleansing and/or disinfectant substance 4 is caused to come out of container 3 and to be consequently sent onto the user's hands (either directly or indirectly). At all events, the cleansing substance sprinkle onto the operating element 2, thereby enabling the cleaning/disinfection operations to be also conveniently carried out on the surgeon's parts that will come into contact with a patient during the operation.

Advantageously, the container can be a hermetically sealed packet 3 made of plastic, metal or generally any other appropriate material, depending on requirements. In this embodiment reduced production costs are obtained, as well as a very small bulkiness and a great facility for use; in addition, due to the presence of packet 3, adoption of a tear tab 6 acting for example on a side of packet 3, is made possible, which tear tab performs the function of the above mentioned means 6 for releasing the cleansing and/or disinfectant substance.

The operating element 2 is formally divided into a housing portion 7 and an active portion 8.

The housing portion 7 essentially houses the container 3 holding substance 4 and enables passage of said substance 4 to the active portion 8 of the cleaning device 1 when the latter is being used. The housing portion 7 essentially consists of a substantially flat support base 9 of any shape; in the embodiment herein show it is of rectangular shape, for example.

The support base 9 has two opposite faces. A first face 10 is brought into contact with the cleansing-substance and/or disinfectant-substance container 3 and preferably container 3 is fastened to said first face of the support base 9 by gluing and similar connecting means; in addition, the second face 11 is opposite to the first face 10 so that the housing portion 7 is connected to the active portion 8 of the operating element 2.

The support base 9 is preferably crossed by a plurality of escape channels 12 (made within the support base 9 itself) giving the cleansing and/or disinfectant substance 4 the possibility of flowing to the active portion 8; advantageously, the escape channels 12 are disposed following a preestablished pattern; for instance, they can be disposed along three parallel axes and in a staggered or alternated relationship with each other. Generally the arrangement pattern of the escape channels 12 will be conveniently studied so as to ensure the greatest possible homogeneity degree in distributing the cleansing and/or disinfectant substance 4 over the active portion 8 of the cleaning device 1.

It is however to be noted that even in the absence of channels 12, the device of the present invention is capable of operating in an efficient manner; in fact, due to actuation of the tear tab 6a, the cleansing/disinfectant substance will come out and exert its function either on the operator's hands or generally on the operator's other parts and/or on the operating element 2 of the cleaning device 1.

In order to improve practical use and operability of device 1, the operating element 2 can be provided with additional holding or confining means 13 extending from the edges of the support base 9 and adapted to keep container 3 at a given position. This additional confining means is particularly useful when a hermetically sealed packet 3 of important volume is wished to be used as the container: in this case these additional confining means 13 inhibits undesired side displacements of container 3 or detrimental side leakages of the cleansing substance 4 true to a wrong positioning of container 3, by substantially acting as additional holding walls with respect to packet 3 which is made of soft plastic material and therefore is not able to keep a fixed position in an autonomous manner.

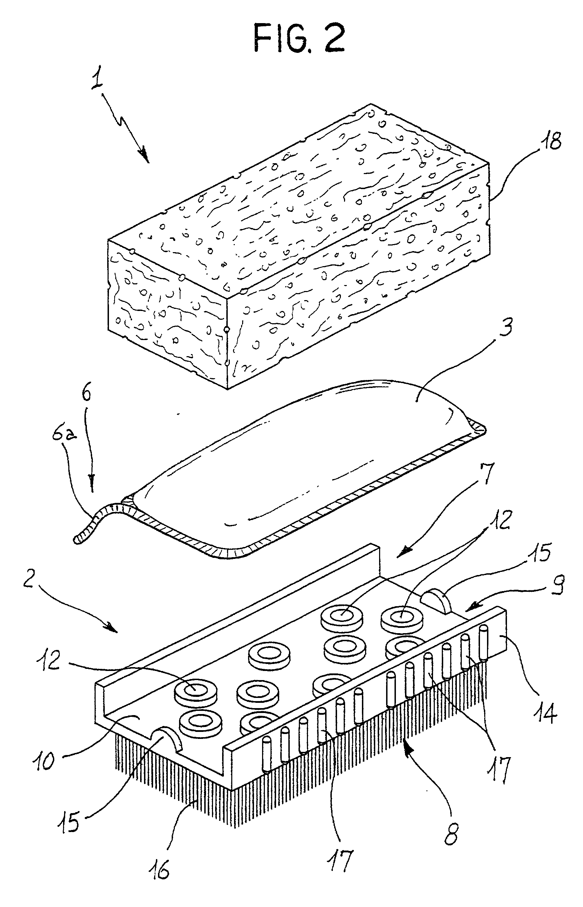

As can be viewed from Fig. 2, this confining means 13 consists of two side wings 14 extending along the longer sides of the support base 9 and of two teeth 15 placed in the middle of the shorter sides of the support base 9.

The active portion 8 comprises a plurality of bristles 16 having a first end 19 substantially linked to the second face 11 of the support base 9. These bristles 16 cover any and every portion of the second face 11 of the support base 9; advantageously the whole second face 11 of the support base 9 is covered with bristles 16, so that the operating capacity of the cleaning device 1 is maximized.

Advantageously, the cleaning device 1 in accordance with the present invention is provided with appropriate grip means 17, consisting of a plurality of projections 17 formed on the side wings 14 and disposed in a substantially vertical orientation. The function of these projections is substantially that of helping the user in maintaining a sure grip on the cleaning device 1 even when his/her hands are wet or covered with protection latex gloves.

Finally, mounted on the operating element 2, by gluing for example, is a block of spongy material 18. Preferably this block of spongy material 18 is made of foamed plastic material. The block of spongy material 18 is disposed on the cleaning device 1 in accordance with the present invention in such a manner that it can receive part of substance 4 at the moment of use and is in the form of a parallelepiped or any other solid, depending on the particular requirements.

The cleaning device 1 is enclosed in sealed packages, a blister pack for example, designed to be used only once. These packages have a predetermined number of cleaning devices 1 inside them; advantageously, each package can contain a single cleaning device 1, so as to ensure the greatest facility for use taking into account its disposable character.

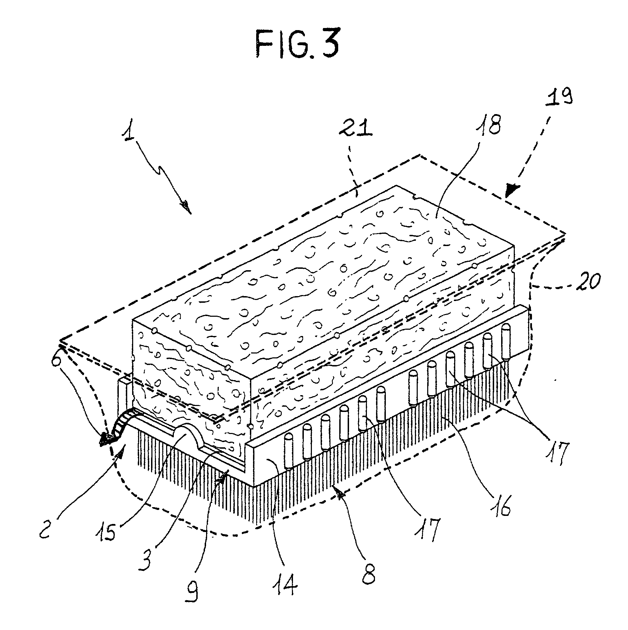

Shown in Fig. 3 is a package 19 in accordance with the invention, designed to house one or more devices 1.

Package 19 comprises a first portion 20, preferably of plastic material and the shape of which matches that of device 1, and a second portion 21 which is substantially flat and is associated with portion 20 thereby forming a hermetically sealed envelope so as to define a hollow space housing device 1. Advantageously the second portion 21 is made of a gas-permeable material. More specifically the second portion 21 can be fully made of gas-permeable material or have at least one portion or window of gas-permeable material.

In the example herein shown the second portion 21 is made of paper material, permeable to gases such as ethylene oxide typically used for sterilization/bacteria elimination operations.

Practically, it is possible to manufacture device 1 and optionally carry out a first sterilization on its parts, then enclose it in the packaging structure 19 and carry out a second sterilization with gases such as ethylene oxide (EO) . It should be noted that use of the second portion 21 of paper material is made possible because the disinfectant or cleansing substance, typically of the liquid type, is sealingly confined within packet 6, a final gas-based sterilization being also feasible, as said, when the product has already been packaged.

As an alternative to the above solution, the first portion 20 can be partly or fully made of paper material.

Use of the cleaning device 1 in accordance with the present invention substantially contemplates the following sequence of steps: after freeing the device 1 from the protection wrapper, the user operates the release means 6 (he/she tears the appropriate tab 6, for example) and, within few seconds, the operating element 2 is sprinkled with the cleansing substance 4. In particular, bristles 16 are reached by the cleansing or disinfectant substance 4, through the escape channels 12, close to the first end 19 thereof; subsequently, by effect of capillarity, the cleansing substance 4 moves forward until bristles 16 are completely wet. Simultaneously, part of the cleansing substance 4 is absorbed by the block 18 made of spongy material which then gives the cleansing substance back when it is rubbed against the parts to be cleaned.

By combining the mechanical action of the operating element 2 (associated with that of the block of spongy material 18, when the latter is present) with the chemical action of substance 4, the user can wash his/her hands with great care, the hygienic quality of the device 1 in his/her possession is ensured because throwaway packages are concerned and the efficiency of the disinfectant action of substance 4 on the operating element 2 is sure because the contact time between said substance and the operating element is sufficient to allow substance 4 to act.

The production process employed for manufacturing the cleaning device 1 in accordance with the present invention is fundamentally based on production of the operating element 2, on parallel production of the block of spongy material 18 and on final assembling between the two cleaning bodies and container 3 holding substance 4; container 3 can be manufactured by a third party, or it can be made simultaneously with the two other pieces.

The above described components can be sterilized before assembling and optionally also after packaging in structure 19, due to the gas-permeability of at least one portion of said structure.

Manufacture of the operating element 2 is essentially based on forming of same by moulding; due to the particular nature of the article in reference, plastic materials having particularly high mechanical features are not required and, as a result of this, the production plant is very simple, which will bring about a reduction in costs.

The block of spongy material 18 is manufactured by moulding as well; in this case appropriate plastic materials are employed that expand within the mould itself, thereby forming a foamed and soft structure, so as to create a synthetic sponge.

Container 3 holding substance 4, a packet 3 for example, is usually made by heat-sealing the edges of two plastic sheets of a substantially rectangular shape. During the heat-sealing step, an injector spout is inserted between the two plastic sheets. This spout enables filling of packet 3 with substance 4 while packet 3 is being formed; when a sufficient amount of substance 4 has been introduced into packet 3, the spout is pulled out and heat-sealing of the end sides of the two plastic sheets is completed.

The final assembling essentially involves gluing or, at all events, mutual engagement (obtained with similar means) between the three previously produced pieces. It is to be noted that gluing is particularly advantageous in terms of construction simplicity and reduced costs.

The invention achieves important advantages.

First of all, due to the presence of container 3 for the disinfectant/cleansing substance 4, a true maintenance of the antiseptic properties of said substance is achieved, since there are no foreign bodies floating within the container 3 itself.

In association with this advantageous feature, also to be considered is the fact that substance 4 within the container 3 is made available to the operator only at the moment it is strictly required for carrying out the cleaning/disinfecting operations. Above all, this substance 4 is made available in a measured amount, thereby avoiding wastes and dispersions of the substance itself.

Another advantage resides in that the great construction simplicity of the present invention allows costs to be greatly reduced, while at the same time use is facilitated to a great degree and a high intrinsic sterility is ensured.

Finally, greatly advantageous is the choice of the above described particular packaging structure 19 allowing gas disinfection and/or sterilization to be carried out even when packaging has been completed.

## Claims

1. A cleaning device (1) comprising:
- an operating element (2) designed to be manipulated by a user and having at least one active portion (8) intended to exert a mechanical action on surfaces to be cleaned;
- at least one container (3) for a cleansing and/or disinfectant substance (4);
characterized in that said container (3) is in engagement with said operating element (2) and is adapted to be brought from a non-operating condition, in which the substance (4) is housed within the container (3) and separated by said operating element (2), to a use condition in which release of the substance (4) from said container (3) is allowed.

2. A device as claimed in claim 1, characterized in that said container (3) comprises at least one hermetically sealed holding wall (5) for retention of the cleansing and/or disinfectant substance (4) inside it.

3. A device as claimed in anyone of the preceding claims, characterised in that said container (3) comprises means (6) for releasing the substance (4) under use conditions of the cleaning device (1), said release means (6) for the substance (4) preferably comprising at least one tear tab (6) acting on at least part of the holding wall (5) of the container (3).

4. A device as claimed in anyone of the preceding claims, characterized in that the container (3) is a hermetically sealed packet (3) holding the substance (4).

5. A device as claimed in anyone of the preceding claims, characterized in that the substance (4) is a disinfectant for medical use.

6. A device as claimed in anyone of the preceding claims, characterized in that the operating element (2) comprises at least one housing portion (7) to receive at least one container (3) for the substance (4), said active portion (8) being connected with said housing portion (7).

7. A device as claimed in claim 6, characterized in that the operating element (2) comprises:
- grip means (17) to enable manipulation of the cleaning device (1) by the user;
- a support base (9) having a first face (10) and a second face (11) opposite to said first face (10), said container (3) for the cleansing substance (4) being disposed at the first face (10) and said active portion (8) extending away from said second face (11); and
- a plurality of escape channels (12) formed in the support base (9) and disposed in a predetermined pattern to allow passage of the cleansing substance (4) from the container (3) to the active portion (8).

8. , A device as claimed in claim 7, characterized in that the housing portion (7) comprises additional holding or confining means (13) extending from the edges of the support base (9) to keep the container (3) for the cleansing substance (4) at a predetermined position.

9. A device as claimed in claim 8, characterized in that said confining means (13) comprises at least two side wings (14) and two teeth (15) disposed on mutually opposite sides of the support base (9)

10. A device as claimed in claim 9, characterized in that said grip means (17) comprises a plurality of projections (17) formed externally of the side wings (14) and disposed in a substantially vertical orientation.

11. A device as claimed in anyone of the preceding claims, characterized in that the active portion (8) comprises a plurality of bristles (16) having a first end (19) linked to the second face (11) of the support base (9) and extending from the opposite side with respect to the container (3), said bristles (16) being sprinkled by the substance (4), under use conditions of the cleaning device (1).

12. A device as claimed in claim 11, characterized in that the bristles (16) extend from at least part of, preferably from the whole surface of, the second face (11) of the support base (9).

13. A device as claimed in claim 11, characterized in that the active portion (8) comprises a block of foamed plastic material (18), preferably in a parallelepiped-shaped spongy configuration and disposed on the opposite side with respect to said bristles (16).

14. A device as claimed in anyone of the preceding claims, characterized in that said container (3) for the substance (4) is rigidly linked to the operating element (2).

15. A device as claimed in claim 14, characterized in that said container (3) for the substance (4) is fastened to the first face (10) of the support base (9).

16. A packaging structure for holding a predetermined number of cleaning devices (1) as claimed in anyone of the preceding claims, said packaging structure comprising at least first and second portions (20, 21) in engagement with each other, at least one of said first and/or second portions having a window made of gas-permeable material.

17. A packaging structure as claimed in claim 16, characterized in that said first portion is made of paper material preferably permeable to an ethylene oxide gas and said second portion is made of plastic material.

18. A structure as claimed in claim 17, characterized in that said first portion is substantially flat and is engaged at its periphery with said second portion the shape of which substantially matches that of said device 1.

19. A process for preparing a packaging structure as claimed in anyone or claims 16 to 18, characterized in that it comprises the following steps:
- setting said first and second portions (20, 21), at least one of said first and second portions having at least one window of gas-permeable material;
- sealingly introducing said cleansing and/or disinfecting substance. (4) into the hermetically sealed packet (6);
- fastening said packet to the operating element (2) to define the device (1);
- preferably carrying out a first sterilization/bacteria elimination operation on said device;
- introducing said device (1) into a hollow space defined by coupling said first and second portions together;
- carrying out a second disinfection/bacteria elimination operation on the packaging structure thus obtained preferably utilising gases such as ethylene oxide.
